# EUROPEAN PATENT APPLICATION

(11) **EP 3 384 823 A1**
(43) Date of publication of application: **10.10.2018**
(21) Application number: 17789329.4
(22) Date of filing: 17.04.2017
(51) Int. Cl.: A61B 1/12

(54) **ENDSCOPE REPROCESSING DEVICE**

(30) Priority: 25.04.2016 JP 2016087183
(71) Applicant: Olympus Corporation, Hachioji-shi Tokyo 192-8507 (JP)
(72) Inventor: TEZUKA, Mitsuyoshi, Hachioji-shi Tokyo 192-8507 (JP); HASEGAWA, Hitoshi, Hachioji-shi Tokyo 192-8507 (JP); IWASAKI, Tomokazu, Hachioji-shi Tokyo 192-8507 (JP); KOGURE, Hisato, Hachioji-shi Tokyo 192-8507 (JP)
(74) Representative: Schicker, Silvia
(86) International application number: PCT/JP2017/015459
(87) International publication number: WO 2017/188044

(57) **Abstract**

An endoscope reprocessing tool 101 includes a connection portion 121 connected to a fluid supply source, a first fluid outlet configured to lead out a fluid, one or a plurality of stands 132 configured to place a distal end portion 41 of an endoscope 1 provided with a guide member configured to guide a treatment instrument to a concave portion 42, a first opening portion 161a and a second opening portion 161b configured to allow the distal end portion 41 to cross, and a positioning portion D configured to hit a proximal end side wall 42c of the concave portion 42 and position the distal end portion 41 when the distal end portion 41 is placed on the stands 132 such that a first opening portion side is on a proximal end and a second opening portion side is on a distal end.

## Description

### Technical Field

The present invention relates to an endoscope reprocessing tool.

### Background Art

Conventionally, there are endoscopes used, for example, in a medical field, provided with a turnable forceps elevator in a concave portion provided at a distal end portion of an insertion portion, in which the forceps elevator allows the forceps to protrude from the concave portion.

International Publication No. 2015/107801 discloses a cleaning tool in which a distal end portion of an insertion portion is butted against a butting portion, the vicinity of the distal end portion of the insertion portion is clamped by a pair of lug portions, the concave portion is positioned with respect to a nozzle and a cleaning liquid is led out from the nozzle into the concave portion.

However, in the conventional cleaning tool, the shape of the butting portion is determined according to the shape of the distal end portion of the insertion portion and the conventional cleaning tool is not applicable to endoscopes with a forceps elevator, the distal end portion of which varies in shape.

It is therefore an object of the present invention to provide an endoscope reprocessing tool capable of positioning a concave portion with respect to a plurality of endoscopes, a distal end portion of an insertion portion of which varies in shape, leading out a fluid from a nozzle into the concave portion and performing reprocessing of an interior of the concave portion.

### Disclosure of Invention

### Means for Solving the Problem

An endoscope reprocessing tool according to an aspect of the present invention includes a connection portion connected to a fluid supply source, a first fluid outlet configured to communicate with the connection portion and lead out a fluid, one or a plurality of stands arranged parallel to a fluid leading-out direction of the first fluid outlet, provided with a guide member to guide a treatment instrument to a concave portion, and configured to place a distal end portion of an endoscope, a first opening portion provided at one end of the stand and configured to allow the distal end portion to cross, a second opening portion provided at the other end of the stand and configured to allow the distal end portion to cross, and a positioning portion configured to hit a proximal end side wall of the concave portion and position the distal end portion when the distal end portion is placed on the stand such that a side of the first opening portion is on a proximal end a side of and the second opening portion is on a distal end.

### Brief Description of the Drawings

Fig. 1 is an explanatory diagram for describing a schematic configuration of an endoscope according to a first embodiment of the present invention;
Fig. 2 is a perspective view of a distal end portion of an insertion portion of the endoscope according to the first embodiment of the present invention;
Fig. 3 is a cross-sectional view of the distal end portion of the insertion portion of the endoscope according to the first embodiment of the present invention;
Fig. 4 is a perspective view illustrating an external appearance configuration of an endoscope reprocessing tool according to the first embodiment of the present invention;
Fig. 5 is a perspective view illustrating an external appearance configuration of the endoscope reprocessing tool according to the first embodiment of the present invention;
Fig. 6 is an explanatory diagram for describing a schematic configuration of an endoscope reprocessor according to the first embodiment of the present invention;
Fig. 7 is an explanatory diagram for describing a state in which the endoscope reprocessing tool according to the first embodiment of the present invention is attached to a holding net disposed on the endoscope reprocessor and the distal end portion of the insertion portion is held by the endoscope reprocessing tool;
Fig. 8 is a cross-sectional view of the distal end portion of the insertion portion of the endoscope and the endoscope reprocessing tool according to the first embodiment of the present invention;
Fig. 9 is a cross-sectional view of the distal end portion of an insertion portion of an endoscope and an endoscope reprocessing tool according to modification 1 of the first embodiment of the present invention;
Fig. 10 is a cross-sectional view of a distal end portion of an insertion portion of an endoscope and an endoscope reprocessing tool according to modification 2 of the first embodiment of the present invention;
Fig. 11 is a perspective view illustrating an external appearance configuration of an endoscope reprocessing tool according to a second embodiment of the present invention;
Fig. 12 is a perspective view illustrating an external appearance configuration of an endoscope reprocessing tool according to the second embodiment of the present invention;
Fig. 13 is an explanatory diagram for describing a state in which the distal end portion of the insertion portion is held by the endoscope reprocessing tool according to the second embodiment of the present invention;
Fig. 14 is an explanatory diagram for describing a state in which the distal end portion of the insertion portion is held by the endoscope reprocessing tool according to the second embodiment of the present invention;
Fig. 15 is an explanatory diagram for describing a state in which the distal end portion provided with an ultrasound transducer is held by the endoscope reprocessing tool according to the second embodiment of the present invention;
Fig. 16 is a perspective view illustrating an external appearance configuration of an endoscope reprocessing tool according to a third embodiment of the present invention;
Fig. 17 is a perspective view illustrating an external appearance configuration of the endoscope reprocessing tool according to the third embodiment of the present invention;
Fig. 18 is a diagram for describing a configuration of a fastener of the endoscope reprocessing tool according to the third embodiment of the present invention;
Fig. 19 is a cross-sectional view of the endoscope reprocessing tool according to the third embodiment of the present invention;
Fig. 20 is an explanatory diagram for describing a state in which the distal end portion of the insertion portion is held by the endoscope reprocessing tool according to the third embodiment of the present invention;
Fig. 21 is an explanatory diagram for describing a state in which the distal end portion with an ultrasound transducer is held by the endoscope reprocessing tool according to the third embodiment of the present invention; and
Fig. 22 is a perspective view illustrating an external appearance configuration of an endoscope reprocessing tool according to a modification of the third embodiment of the present invention.

### Best Mode for Carrying Out the Invention

Hereinafter, embodiments of the present invention will be described with reference to the accompanying drawings.

### (First Embodiment)

### (Configuration)

Fist, an endoscope 1 will be described.

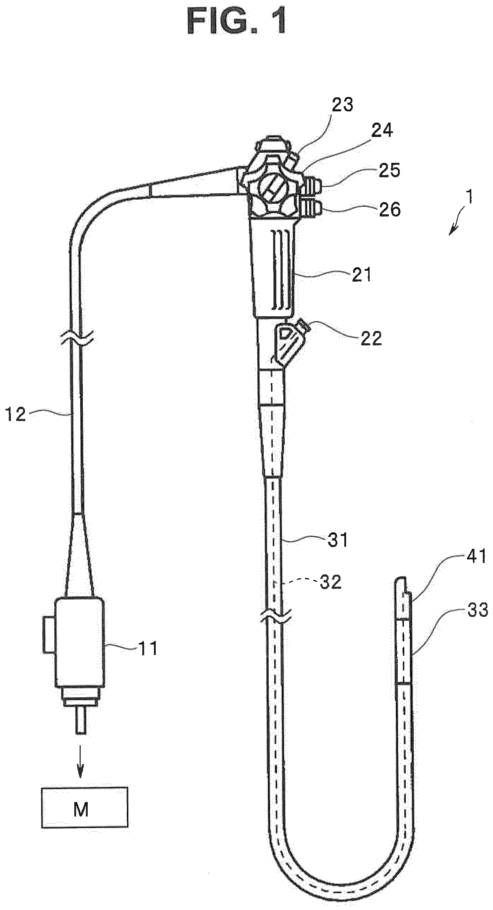

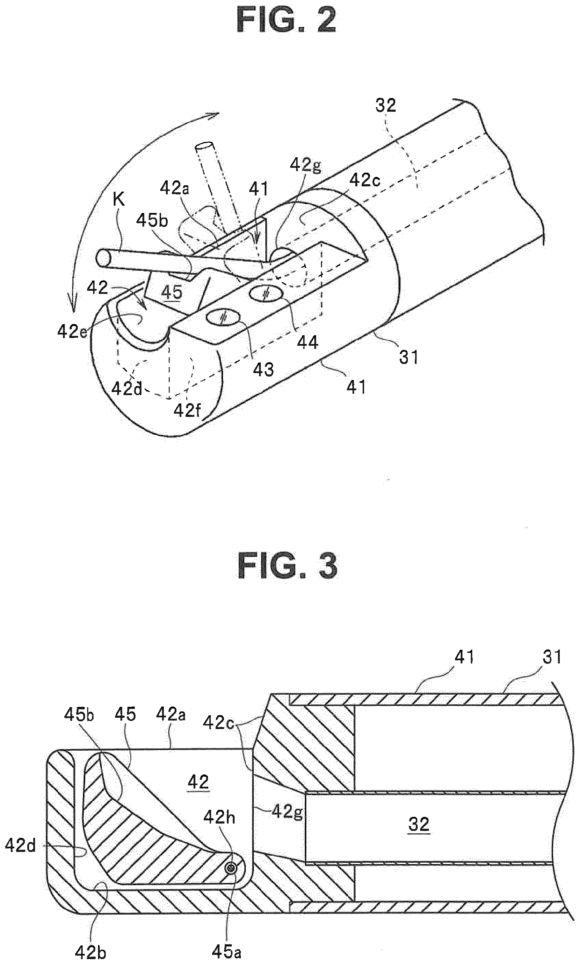

Fig. 1 is an explanatory diagram for describing a schematic configuration of the endoscope 1 according to a first embodiment of the present invention. Fig. 2 is a perspective view of a distal end portion 41 of an insertion portion 31 of the endoscope 1 according to the first embodiment of the present invention. Fig. 3 is a cross-sectional view of the distal end portion 41 of the insertion portion 31 of the endoscope 1 according to the first embodiment of the present invention.

The endoscope 1 includes a connector 11, an operation portion 21 and the insertion portion 31.

The connector 11 is configured to be connectable to an endoscope apparatus body M. The connector 11 is connected to the operation portion 21 via a universal cord 12. The endoscope 1 is configured to receive illumination light for illuminating a subject from a light source of the endoscope apparatus body M via the connector 11 and output an image pickup signal of the subject picked up by the endoscope 1 to the endoscope apparatus body M.

The operation portion 21 is configured to be able to perform various types of operations of the endoscope 1. The operation portion 21 includes a forceps insertion port 22, a forceps raising lever 23, an angle knob 24, a suction button 25 and an air/water feeding button 26. The forceps insertion port 22 is configured to communicate with a forceps insertion passage 32 and allow the insertion of a forceps K (Fig. 2) to protrude from the distal end portion 41 of the insertion portion 31. The forceps raising lever 23 is connected to a forceps elevator 45 of the distal end portion 41 via a wire which is not shown and can raise or lay down the forceps elevator 45 through turning operation. The angle knob 24 is connected to a bending portion 33 of the insertion portion 31 via a wire which is not shown and can bend the bending portion 33 through turning operation. The suction button 25 can suction a suction object from the distal end portion 41 through button operation. The air/water feeding button 26 can send a fluid from a fluid feeding section which is not shown provided at the distal end portion 41 through button operation.

The insertion portion 31 is formed into an elongated shape and configured to be insertable into the subject. The insertion portion 31 includes the bending portion 33 configured to be bent by the angle knob 24.

As shown in Fig. 2, the distal end portion 41 of the insertion portion 31 includes a concave portion 42, an illumination section 43 and an image pickup section 44.

The concave portion 42 includes the forceps elevator 45 and is configured to allow the forceps K inserted from the forceps insertion port 22 to protrude. The concave portion 42 includes an opening 42a, a base wall 42b, a proximal end side wall 42c formed so as to erect from the base wall 42b, a distal end side wall 42d and both left and right side walls 42e and 42f. The opening 42a is formed on a circumference of the distal end portion 41. Each of both the left and right side walls 42e and 42f is formed so as to neighbor the proximal end side wall 42c and the distal end side wall 42d. The proximal end side wall 42c is formed from the base wall 42b to the outer circumference of the insertion portion 31 and includes a forceps outlet 42g configured to communicate with the forceps insertion passage 32 in the proximal end side wall 42c. Both the left and right side walls 42e and 42f are each provided with a rotary shaft 42h configured to pivotally support the forceps elevator 45 (Fig. 3). The forceps elevator 45 is turnably attached to the rotary shaft 42h through a rotary shaft hole 45a of the proximal end portion. The forceps elevator 45 is attached to a wire connected to the forceps raising lever 23 at the distal end side of the rotary shaft 42h and configured to be turnable in a raising direction toward the opening 42a or in a laying-down direction toward the base wall 42b through the turning operation of the forceps raising lever 23. The forceps elevator 45 includes a guide surface 45b formed into a concave curved shape at a position facing the forceps outlet 42g. When the forceps K is inserted from the forceps insertion port 22, the forceps K is led out from the forceps outlet 42g and the forceps elevator 45 is turned in the raising direction, the forceps K is guided along the guide surface 45b and made to protrude from the opening 42a.

That is, the forceps elevator 45 is a guide member configured to guide the forceps K which is a treatment instrument.

The illumination section 43 is configured to be able to illuminate the subject with illumination light guided from a light source of the endoscope apparatus body M via the connector 11.

The image pickup section 44 includes a photoelectric conversion element such as a CCD. The image pickup section 44 is configured to receive reflected light of the subject illuminated by the illumination section 43, photoelectrically convert the reflected light and output an image pickup signal to the endoscope apparatus body M via the connector 11.

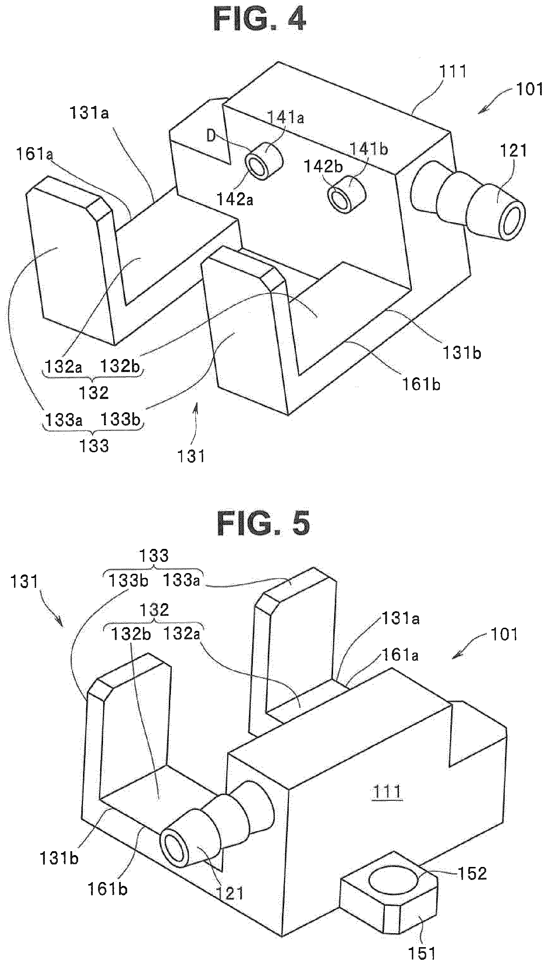

Fig. 4 and Fig. 5 are perspective views illustrating an external appearance configuration of an endoscope reprocessing tool 101 according to the first embodiment of the present invention.

Next, the endoscope reprocessing tool 101 will be described.

The endoscope reprocessing tool 101 is configured to be able to lead out a fluid such as a cleaning liquid to a predetermined position in the concave portion 42 of the insertion portion 31 of the endoscope 1 and perform reprocessing of the endoscope. The endoscope reprocessing tool 101 includes a body section 111, a connection portion 121, a holding portion 131, a first nozzle 141a, a second nozzle 141b and a positioning portion D.

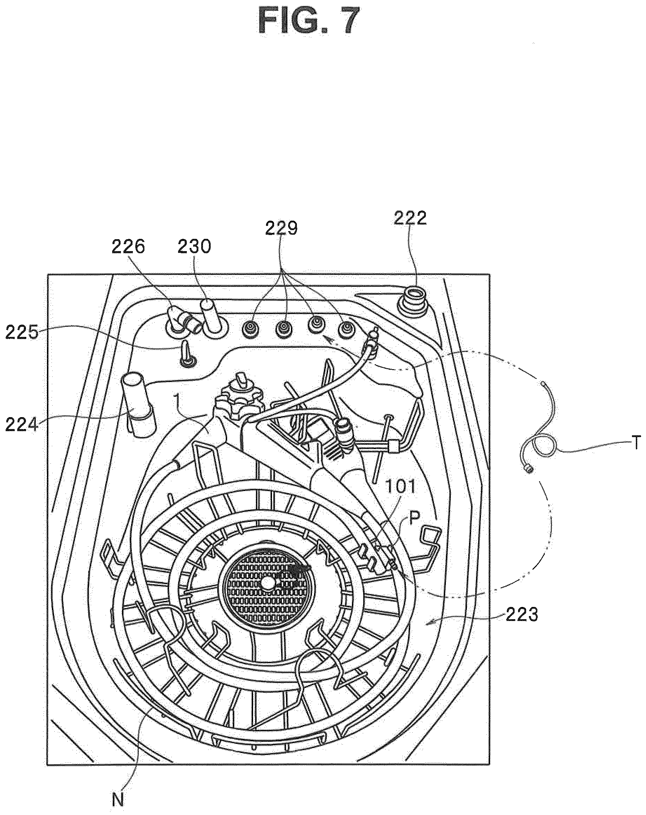

The body section 111 is formed into a block shape. The body section 111 is provided with a fixing portion 151. The fixing portion 151 includes a mounting hole 152 for the endoscope reprocessing tool 101 to be mounted on a holding net N (Fig. 7). The body section 111 includes a fluid channel R inside to be caused to communicate with the connection portion 121, the first nozzle 141a and the second nozzle 141b.

The connection portion 121 is configured to be connectable to an endoscope reprocessor 201 which is a fluid supply source, which will be described later. The connection portion 121 protrudes from the body section 111 and is formed into a cylindrical shape. The connection portion 121 includes the fluid channel R communicating with the body section 111 on an inner circumferential side. The connection portion 121 includes a slip preventing protrusion for preventing slippage of an externally fitted connection tube T on the outer circumference. The connection portion 121 is connected to the endoscope reprocessor 201 via the connection tube T and the endoscope reprocessor 201 introduces a fluid into the connection portion 121. In Fig. 4 and Fig. 5, the connection portion 121 is provided on a side of the body section 111.

The holding portion 131 is configured to be able to hold the distal end portion 41. The holding portion 131 includes stands 132, stopper portions 133, a first opening portion 161a and a second opening portion 161b.

The stand 132 is disposed parallel to a fluid leading-out direction of a first fluid outlet 142a which will be described later and configured to be able to place the distal end portion 41 of the endoscope 1, with a guide member for guiding a treatment instrument provided in the concave portion 42. The number of the stands 132 is not particularly limited, and, for example, as shown by reference numerals 132a and 132b in Fig. 5, the stands 132 may be formed so as to respectively extend from a base part on the first nozzle 141a side and a base part on the second nozzle 141b side of the body section 111 in a direction along the fluid leading-out direction of the first fluid outlet 142a.

The stopper portion 133 is configured to be able to stop the distal end portion 41 so as to prevent the distal end portion 41 placed on the stand 132 from coming off. In Fig. 5, the stopper portions 133a and 133b are formed on 132a and 132b constituting the stands 132. The stopper portions 133a and 133b are provided on the stands 132a and 132b so as to cross the fluid leading-out direction of the first fluid outlet 142a so as to face the body section 111 at a predetermined distance from the body section 111. A crossing angle of the stopper portion 133 with respect to the fluid leading-out direction is not particularly limited, but can be set to, for example, 90 degrees.

The first opening portion 161a is provided at one end of the stand 132 in a direction orthogonal to the fluid leading-out direction of the first fluid outlet 142a and is configured to allow the distal end portion 41 to cross. More specifically, the first opening portion 161a is formed to be surrounded by the body section 111, the stand 132a and the stopper portion 133a on three sides to allow the distal end portion 41 to be placed in a first nozzle side end portion 131a of the holding portion 131 so as to open the space surrounded by the body section 111, the stand 132a and the stopper portion 133a.

The second opening portion 161b is provided at the other end of the stand 132 and is configured so as to allow the distal end portion 41 to cross. More specifically, the second opening portion 161b is formed to be surrounded by the body section 111, the stand 132b and the stopper portion 133b on three sides to allow the distal end portion 41 to be crossly placed in a second nozzle side end portion 131b of the holding portion 131 so as to open the space surrounded by the body section 111, the stand 132b and the stopper portion 133b.

The first nozzle 141a is configured to be able to lead out the fluid introduced from the connection portion 121. The first nozzle 141a protrudes from the body section 111 and is formed into a cylindrical shape. The first nozzle 141a includes the fluid channel R communicating with the body section 111 on an inner circumferential side. The first nozzle 141a includes a first fluid outlet 142a.

The first fluid outlet 142a communicates with the connection portion 121 and is configured to be able to lead out the fluid. More specifically, the first fluid outlet 142a is formed at a distal end of the first nozzle 141a, communicates with the fluid channel R in the connection portion 121 via the fluid channel R in the first nozzle 141a and the fluid channel R in the body section 111 to lead out the fluid introduced into the connection portion 121.

The second nozzle 141b is configured to be able to lead out the fluid introduced from the connection portion 121. The second nozzle 141b protrudes from the body section 111 and is formed into a cylindrical shape. The second nozzle 141b includes the fluid channel R communicating with the body section 111 on the inner circumferential side. The second nozzle 141b includes a second fluid outlet 142b.

The second fluid outlet 142b is disposed closer to the second opening portion 161b side than the first fluid outlet 142a and configured to be able to lead out the fluid. More specifically, the second fluid outlet 142b is formed at a distal end of the second nozzle 141b, communicates with the fluid channel R in the connection portion 121 via the fluid channel R in the second nozzle 141b and the fluid channel R in the body section 111 to lead out the fluid introduced into the connection portion 121.

When the distal end portion 41 is placed on the stand 132 so that the first opening portion 161a side is on the proximal end and the second opening portion 161b side is on the distal end, the positioning portion D hits the proximal end side wall 42c of the concave portion 42 and thereby positions the distal end portion 41. In the first embodiment, the first nozzle 141a constitutes the positioning portion D.

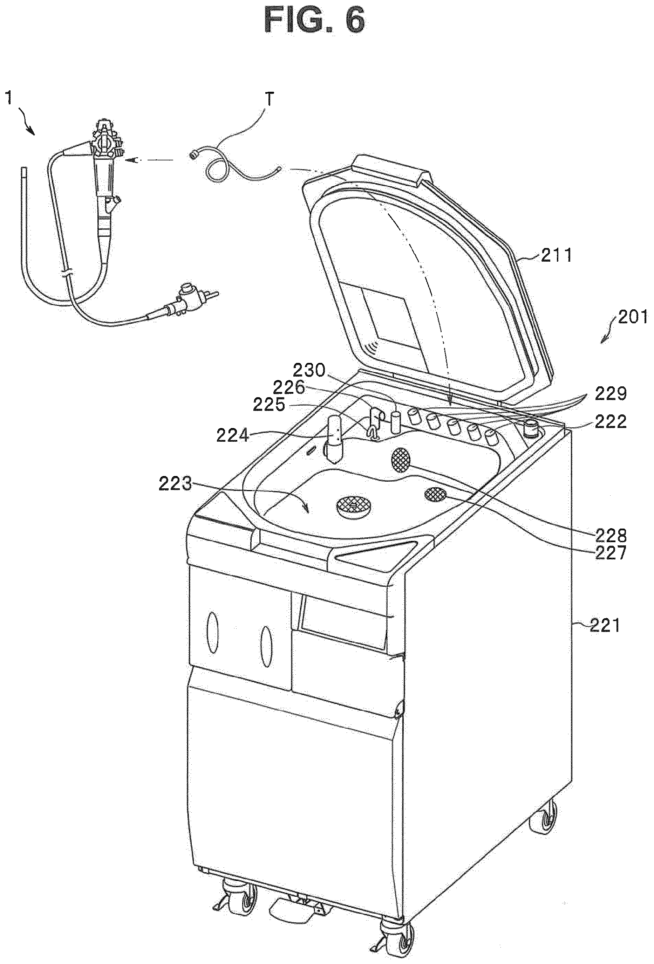

Fig. 6 is an explanatory diagram for describing a schematic configuration of the endoscope reprocessor 201 according to the first embodiment of the present invention. Fig. 7 is an explanatory diagram for describing a state in which the endoscope reprocessing tool 101 according to the first embodiment of the present invention is attached to a holding net N disposed on the endoscope reprocessor 201 and the distal end portion 41 of the insertion portion 31 is held by the endoscope reprocessing tool 101.

Next, a schematic configuration of the endoscope reprocessor 201 will be described.

The endoscope reprocessor 201 performs reprocessing of the contaminated endoscope 1, parts or accessories or the like of the endoscope 1. The reprocessing referred to here is not particularly limited, but may be any one of rinsing with water, cleaning of washing off dirt such as organic matter, disinfection of disabling specified microbes, sterilization of eliminating or annihilating all microbes or a combination of these treatments. The accessories are not particularly limited and examples of the accessories include the suction button 25 and the air/water feeding button 26 which are attached to the endoscope 1 when used and removed from the endoscope 1 during reprocessing and a distal end cover for covering the distal end portion 41 of the endoscope 1.

As shown in Fig. 6, the endoscope reprocessor 201 includes a top cover 211 and an apparatus body 221. The apparatus body 221 includes a water supply hose connection portion 222, which is connected to external water supply means.

The top cover 211 is freely openably/closably provided for a treatment tank 223 so as to cover the treatment tank 223 and when the top cover 211 is opened, the interior of the treatment tank 223 is exposed to the outside.

In Fig. 6, the treatment tank 223 is formed into a tub shape so as to allow the endoscope 1 to be placed in the treatment tank 223, but the shape of the treatment tank 223 is not limited to this. The treatment tank 223 includes a water level sensor 224, a detergent nozzle 225, a medicinal solution inlet 226, a drain port 227, a circulation port 228, connectors 229 and a circulation nozzle 230.

The endoscope reprocessor 201 is connected to the endoscope 1 via the connection tube T. Note that the endoscope reprocessor 201 may be structured to be directly connected without the connection tube T.

As shown in Fig. 7, the holding net N configured to hold the endoscope 1 connected to the endoscope reprocessor 201 is placed in the treatment tank 223 of the endoscope reprocessor 201. The mounting hole 152 of the fixing portion 151 is attached to a pin P of the holding net N and the endoscope reprocessing tool 101 is placed in the holding net N such that the fixing portion 151 is oriented toward the outer circumference of the holding net N and the holding portion 131 is oriented toward the center of the holding net N. The endoscope reprocessing tool 101 and the endoscope reprocessor 201 are connected together by attaching one end of the connection tube T to the connection portion 121 of the endoscope reprocessing tool 101 and attaching the other end of the connection tube T to the connector 229 of the endoscope reprocessor 201.

Note that the connection tube T may be attached to a special tube connector which is different from the endoscope tube connection connector of the connectors 229 to allow the endoscope reprocessing tool 101 to lead out the fluid with greater force.

The endoscope 1 is wound and held in the holding net N. The distal end portion 41 is held by the holding portion 131 such that the opening 42a of the concave portion 42 is oriented toward the body section 111. The insertion portion 31 is biased in an outer circumferential direction of the holding net N in which the body section 111 is placed by a restoring force trying to return to a linear state.

### (Operation)

Next, operation of the endoscope reprocessing tool 101 will be described.

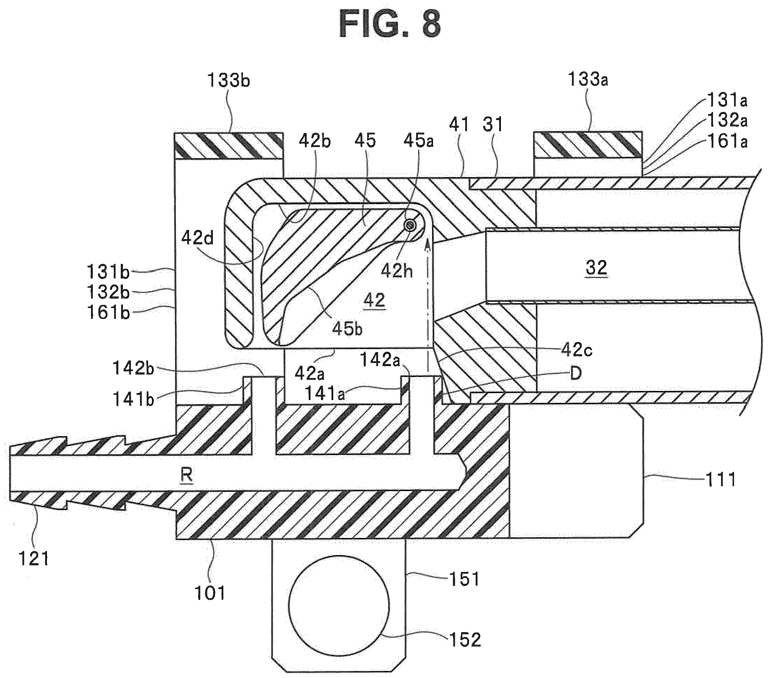

Fig. 8 is a cross-sectional view of the distal end portion 41 of the endoscope 1 and the endoscope reprocessing tool 101 according to the first embodiment of the present invention.

As shown in Fig. 8, the endoscope 1 is placed on the stands 132a and 132b of the endoscope reprocessing tool 101 such that the first opening portion 161a side is on the proximal end and the second opening portion 161b side is on the distal end. In other words, the endoscope reprocessing tool 101 holds the distal end portion 41 by the holding portion 131 such that the proximal end side of the distal end portion 41 is disposed on the first nozzle 141a side and the distal end side of the distal end portion 41 is disposed on the second nozzle 141b side.

The outer circumference of the first nozzle 141a which is the positioning portion D hits the proximal end side wall 42c of the concave portion 42 and the distal end portion 41 is positioned.

When the distal end portion 41 is positioned, the first nozzle 141a is disposed adjacent to the proximal end side wall 42c so as to be able to lead out a fluid into a gap between the proximal end side wall 42c and the forceps elevator 45 (single-dot dashed line in Fig. 8). On the other hand, the second nozzle 141b is disposed to face the concave portion 42 so as to be able to lead out the fluid into the concave portion 42.

When the endoscope reprocessor 201 leads out the fluid from the connector 229, the fluid is introduced into the connection portion 121 via the connection tube T. The fluid introduced into the connection portion 121 is led out from the first nozzle 141a and the second nozzle 141b via the fluid channel R in the body section 111. When pushed by the introduced fluid, the distal end portion 41 is butted by the stopper portions 133a and 133b.

The fluid led out from the first nozzle 141a and the second nozzle 141b flows into the concave portion 42 to perform reprocessing. Part of the fluid led out from the first nozzle 141a passes through the gap between the forceps elevator 45 and the proximal end side wall 42c, reaches the base wall 42b, also flows into the gap between the forceps elevator 45 and the proximal end side wall 42c, the gap between the rotary shaft hole 45a and the rotary shaft 42h and the back side of the forceps elevator 45 to perform reprocessing.

In the endoscope reprocessing tool 101, the positioning portion D hits the proximal end side wall 42c of the concave portion 42 and positions the distal end portion 41. The distal end portion 41 can be positioned in this way even for the endoscope 1 where the concave portion 42 is formed to be long.

In the endoscope reprocessing tool 101, the first opening portion 161a and the second opening portion 161b are opened so that the insertion portion 31 may cross. For example, when the endoscope 1 is an ultrasonic scope, the distal end portion 41 of which includes an ultrasonic transducer H, or the like, the distal end portion 41 can be positioned even in the case where the distal end portion 41 is formed to be long.

The endoscope reprocessing tool 101 positions and holds the distal end portion 41, causes the first fluid outlet 142a to lead out the fluid toward the gap between the forceps elevator 45 and the proximal end side wall 42c. It is thereby possible to perform reprocessing of the interior of the concave portion 42 with the fluid strongly and effectively.

The endoscope reprocessing tool 101 is formed with a simple structure. This allows the endoscope reprocessing tool 101 to be manufactured at low cost.

According to the above-described first embodiment, for a plurality of endoscopes 1, the distal end portions 41 of the insertion portion 31 of which varies in shape, the endoscope reprocessing tool 101 positions the concave portion 42, leads out the fluid from the first nozzle 141a and the second nozzle 141b into the concave portion 42, and can thereby perform reprocessing of the interior of the concave portion 42.

### (Modification 1 of First Embodiment)

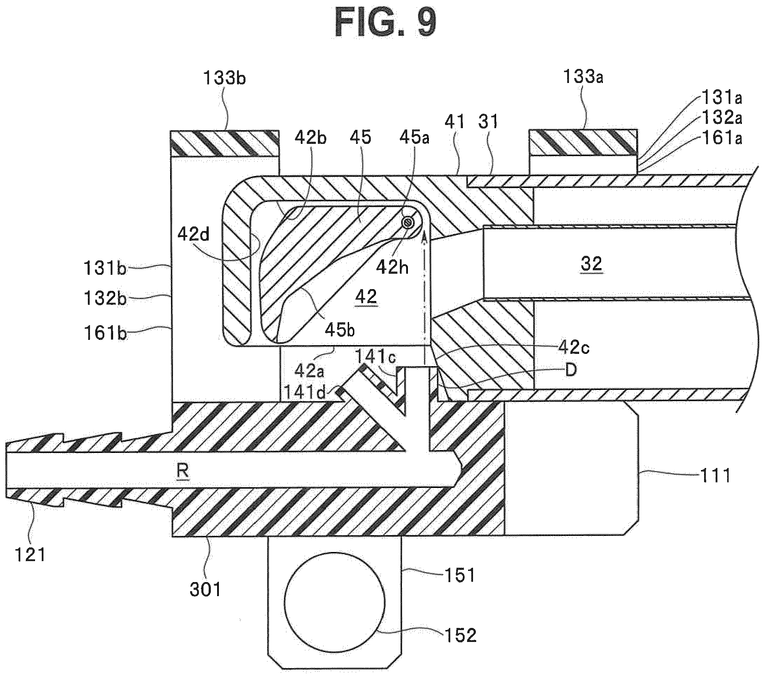

Fig. 9 is a cross-sectional view of the distal end portion 41 of the insertion portion 31 of the endoscope 1 and an endoscope reprocessing tool 301 according to modification 1 of the first embodiment of the present invention.

In the first embodiment, the positioning portion D includes the first nozzle 141a that can lead out the fluid in one direction, but the positioning portion D may also include a first nozzle 141c and a second nozzle 141d that can lead out the fluid in two directions.

In the endoscope reprocessing tool 301, the second nozzle 141d is formed so as to branch from the proximal end of the first nozzle 141c and protrude diagonally from the proximal end of the first nozzle 141c.

That is, in the endoscope reprocessing tool 301, the positioning portion D includes the first nozzle 141c and the second nozzle 141d that can lead out the fluid in two directions.

### (Modification 2 of First Embodiment)

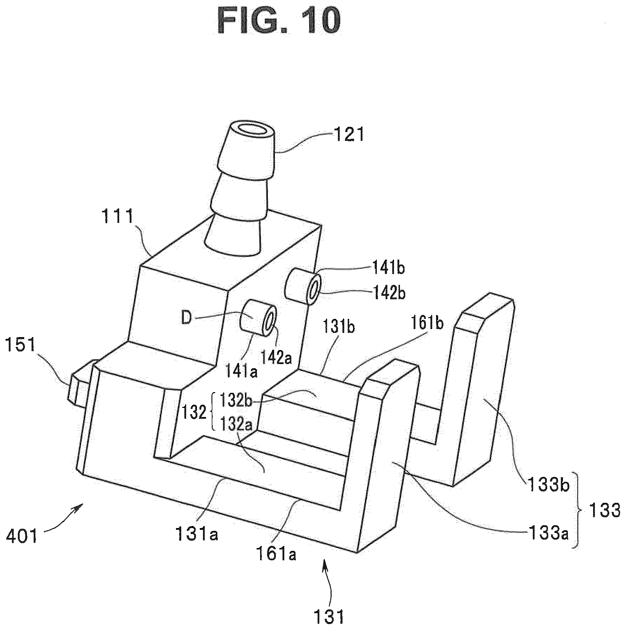

Fig. 10 is a cross-sectional view of the distal end portion 41 of the insertion portion 31 of the endoscope 1 and an endoscope reprocessing tool 401 according to modification 2 of the first embodiment of the present invention.

In the first embodiment, the connection portion 121 is provided on a side of the body section 111, but the connection portion 121 may be provided at the top of the body section 111.

In the endoscope reprocessing tool 401, the connection portion 121 is provided at the top of the body section 111. Note that the connection portion 121 is not limited to being provided at the side or top of the body section 111, but may be provided at any location of the body section 111 though not shown.

### (Second Embodiment)

In the first embodiment and modifications 1 and 2 of the first embodiment, the holding portion 131 is not opened/closed, but the holding portion 131 may be configured to be opened/closed.

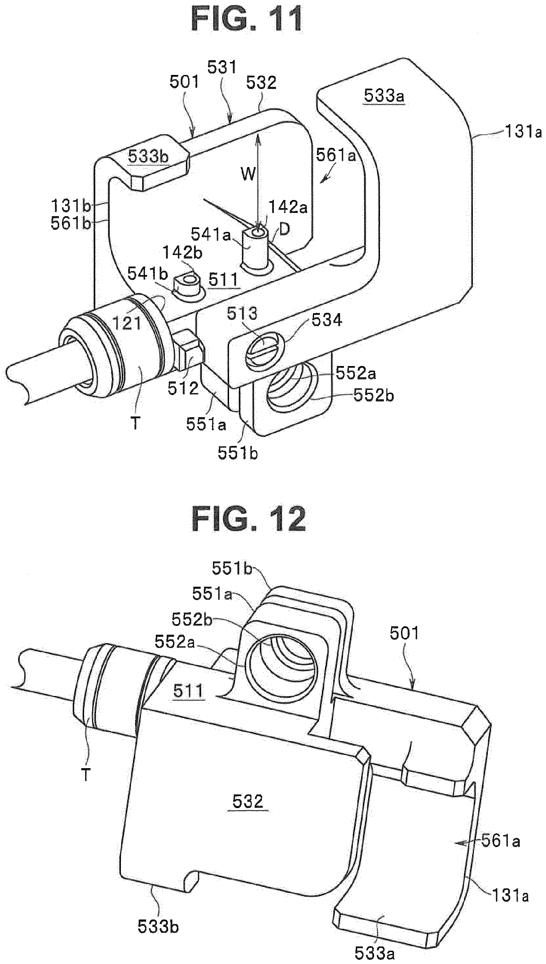

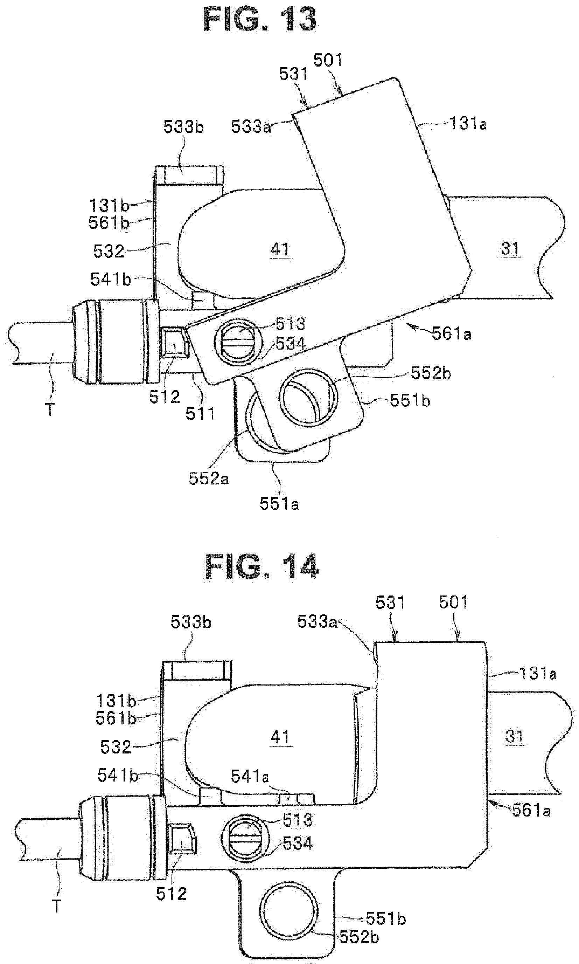

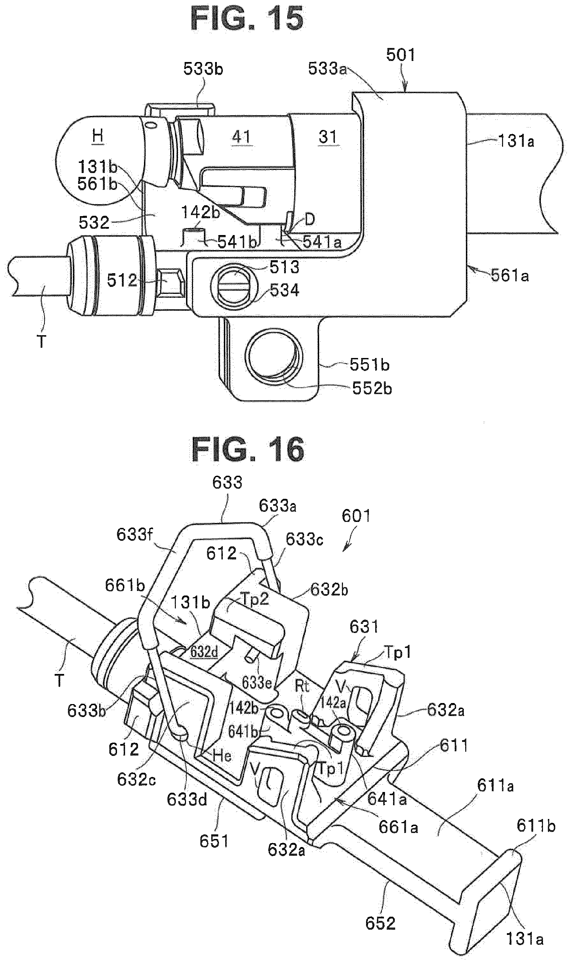

Fig. 11 and Fig. 12 are perspective views illustrating an external appearance configuration of an endoscope reprocessing tool 501 according to the second embodiment of the present invention. Fig. 13 and Fig. 14 are explanatory diagrams for describing a state in which the distal end portion 41 of the insertion portion 31 is held by the endoscope reprocessing tool 501 according to the second embodiment of the present invention. Fig. 15 is an explanatory diagram for describing a state in which the distal end portion 41 with the ultrasound transducer H is held by the endoscope reprocessing tool 501 according to the second embodiment of the present invention. In Fig. 11 to Fig. 15, the tube T is attached to the connection portion 121. In the description of the present embodiment, description of the same components as the components in the other embodiments and modifications will be omitted.

The endoscope reprocessing tool 501 includes a body section 511, a holding portion 531, a first nozzle 541a, a second nozzle 541b and fixing portions 551a and 551b.

As shown in Fig. 11, the body section 511 is formed into a block shape. The body section 511 includes a restricting protrusion portion 512 and a shaft portion 513.

The restricting protrusion portion 512 is formed so as to protrude from the body section 511. The restricting protrusion portion 512 is provided at a position at which the restricting protrusion portion 512 is hit by a stopper portion 533a which will be described later through turning and configured to restrict the turning range of the stopper portion 533a.

The shaft portion 513 is formed so as to protrude from the body section 511. The shaft portion 513 includes a locking claw for dropout prevention at a distal end of the shaft portion 513.

As shown in Fig. 11 and Fig. 12, the holding portion 531 includes a stand 532 and stopper portions 533a and 533b.

The stand 532 is formed so as to extend from the body section 511 in a direction along the fluid leading-out direction to allow the distal end portion 41 of the insertion portion 31 to be placed.

The stopper portions 533a and 533b are configured to be able to stop the distal end portion 41 so as to prevent the distal end portion 41 placed on the stand 532 from coming off.

The stopper portion 533a is freely turnably supported by the body section 511. The stopper portion 533a includes a bearing hole 534 and is attached to the body section 511 by inserting the bearing hole 534 around the shaft portion 513 and locking the stopper portion 533a with a locking claw. The stopper portion 533a is formed into a block shape along a direction from a second nozzle side end portion 131b to a first nozzle side end portion 131a, then the first nozzle 541a side is formed so as to extend along the fluid leading-out direction and then the extending portion is formed to bend in a direction crossing the direction along the fluid leading-out direction.

The stopper portion 533b is formed such that the second nozzle 541b side of the stand 532 is bent in a direction crossing the direction along the fluid leading-out direction.

The first nozzle 541a and the second nozzle 541b are configured to be able to lead out a fluid introduced from the connection portion 121. The first nozzle 541a is formed to have a predetermined length which is longer than the second nozzle 541b so as to reliably position the distal end portion 41 and allow the fluid to be sent deeply into the concave portion 42. The predetermined length is set such that a gap width W from the distal end of the first nozzle 541a to the stopper portion 533a and 533b becomes smaller than the diameter of the endoscope 1. Note that the predetermined length may also be set such that the gap width W becomes larger than the diameter of the endoscope 1.

The fixing portions 551a and 551b are provided so as to protrude toward a direction opposite to the fluid leading-out direction. The fixing portion 551a is provided on the body section 511. The fixing portion 551a includes a mounting hole 552a for mounting on the holding net N. The fixing portion 551b is provided on the stopper portion 533a. The fixing portion 551b includes a mounting hole 552b. The mounting holes 552a and 552b face each other to allow the holding pin P to pass through the holes when the stopper portion 533a is kept in the closed state. When the holding pin P is passed through the mounting holes 552a and 552b, the stopper portion 533a is locked so as to be kept in a closed state.

A first opening portion 561a is provided at one end of the stand 532 in a direction orthogonal to the fluid leading-out direction of the first fluid outlet 142a and configured to allow the distal end portion 41 to cross. More specifically, the first opening portion 561a is formed to be surrounded on three sides by the body section 511, the stand 532 and the stopper portion 533a to allow the distal end portion 41 to be placed on the first nozzle side end portion 131a, and to open the space surrounded by the body section 511, the stand 532 and the stopper portion 533a.

A second opening portion 561b is provided at the other end of the stand 532 and configured to allow the distal end portion 41 to cross. More specifically, the second opening portion 561b is formed to be surrounded on three sides by the body section 511, the stand 532 and the stopper portion 533b on three sides to allow the distal end portion 41 to be crossly placed on the second nozzle side end portion 131b of the holding portion 531 and to open the space surrounded by the body section 511, the stand 532 and the stopper portion 533b.

As shown in Fig. 13, the stopper portion 533a can be placed in an open state through turning. As shown in Fig. 14, the stopper portion 533a can be placed in a closed state through turning. After placing the stopper portion 533a in an open state, positioning the concave portion 42 through the positioning portion D and placing the distal end portion 41 on the stopper portion 533a, the stopper portion 533a is placed in a closed state, the holding pin P is passed through the mounting holes 552a and 552b and the holding portion 531 holds the distal end portion 41.

As shown in Fig. 15, by crossly placing the holding portion 531 on the second opening portion 561b, the holding portion 531 can also hold the distal end portion 41 with the ultrasound transducer H.

That is, the stopper portion 533a is freely openable/closable with respect to the stand 532. More specifically, the stopper portion 533a is freely turnable around the shaft portion 513 provided in a direction crossing the direction along the fluid leading-out direction.

According to the above-described second embodiment, the endoscope reprocessing tool 501 can open/close the holding portion 531, position the concave portion 42 with respect to a plurality of endoscopes 1, the distal end portion 41 of the insertion portion 31 of which varies in shape, lead out a fluid from the first nozzle 541a and the second nozzle 541b into the concave portion 42 and perform reprocessing of the interior of the concave portion 42.

### (Third Embodiment)

In the first embodiment and modifications 1 and 2 of the first embodiment, and the second embodiment, the distal end portion 41 is stopped by the stopper portions 133, 533a and 533b, but the distal end portion 41 may be configured to be stopped by a fastener 633.

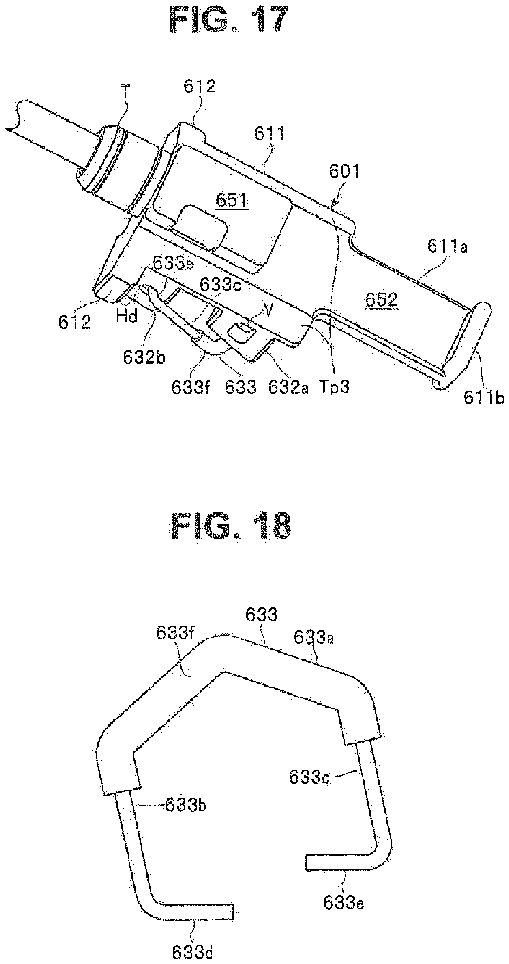

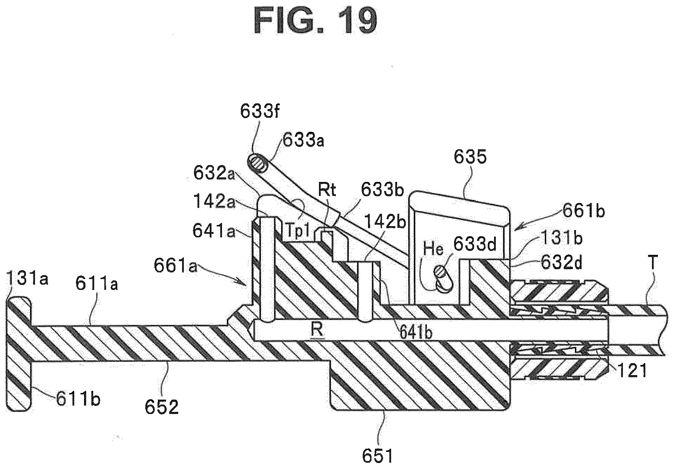

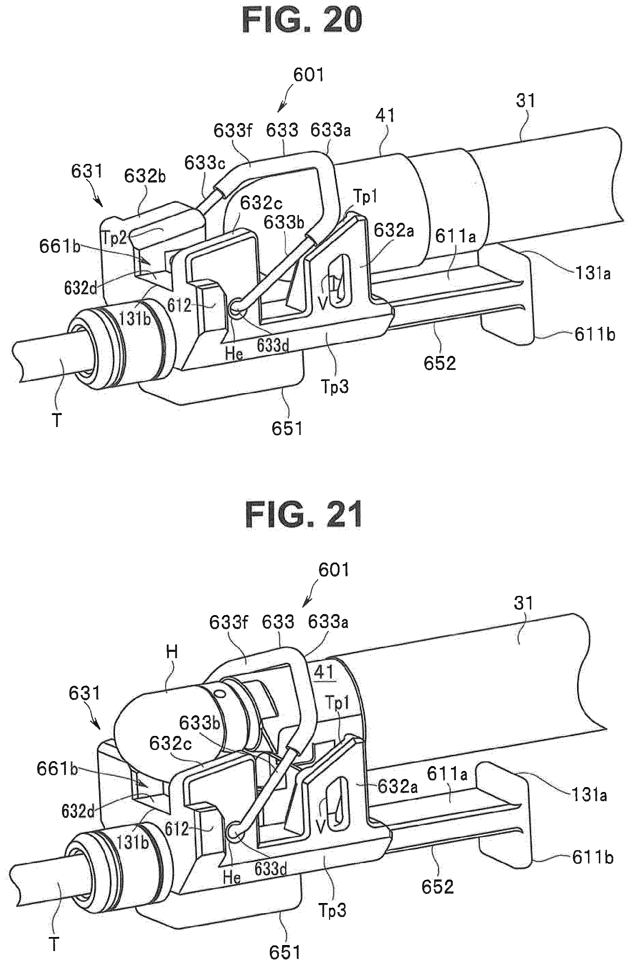

Fig. 16 and Fig. 17 are perspective views illustrating an external appearance configuration of an endoscope reprocessing tool 601 according to a third embodiment of the present invention. Fig. 18 is a diagram for describing a configuration of a fastener 633 of the endoscope reprocessing tool 601 according to the third embodiment of the present invention. Fig. 19 is a cross-sectional view of the endoscope reprocessing tool 601 according to the third embodiment of the present invention. Fig. 20 is an explanatory diagram for describing a state in which the distal end portion 41 of the insertion portion 31 is held by the endoscope reprocessing tool 601 according to the third embodiment of the present invention. Fig. 21 is an explanatory diagram for describing a state in which the distal end portion 41 with the ultrasound transducer H is held by the endoscope reprocessing tool 601 according to the third embodiment of the present invention. In the present embodiment, description of the same components as the components in the other embodiments and modifications will be omitted.

The endoscope reprocessing tool 601 includes a body section 611, a holding portion 631, a first nozzle 641a and a second nozzle 641b.

As shown in Fig. 16, the body section 611 is formed into a tabular shape. The body section 611 includes an extending piece 611a and a restriction protruding section 612.

The extending piece 611a is formed so as to extend from the body section 611 in a direction from a second nozzle 641b toward a first nozzle 641a. A support piece 611b configured to support the distal end portion 41 is provided at an end portion of the extending piece 611a so as to cross the extending direction of the extending piece 611a.

The restriction protruding section 612 is formed so as to protrude from the body section 611. The restriction protruding section 612 is provided at a position at which the restriction protruding section 612 is hit by the fastener 633 which will be described later through turning and configured to restrict a turning range of the fastener 633.

As shown in Fig. 16 and Fig. 17, the holding portion 631 includes stands 632a, 632b, 632c and 632d, and the fastener 633.

The stands 632a are provided at both corners closer to the first nozzle 641a side than the second nozzle 641b of the body section 611 so as to face each other in order to be able to place the distal end portion 41 of the insertion portion 31 on the stands 632a. The stands 632a are formed so as to protrude from the body section 611. The stands 632a have tapered portions Tp1 inclined so as to follow the laid-down fastener 633 at distal ends in order to be able to receive the fastener 633. The stands 632a have relief holes V to release the fluid on the stands 632a at the center.

The stands 632b and 632c are provided at both corners closer to the second nozzle 641b side than the first nozzles 641a of the body section 611 so as to face each other. The stands 632b and 632c are formed so as to protrude from the body section 611. The stands 632b and 632c have tapered portions Tp2 inclined so as to hit the distal end portion 41 at the top of the surfaces facing each other so as to be able to receive the distal end portion 41. The stand 632c includes a bearing hole He. The stand 632b includes a bearing hole Hd (Fig. 17). The bearing holes Hd and He are formed at positions at the same distance from the second nozzle side end portion 131b and at positions at different distances from the respective proximal ends of the stands 632b and 632c. In the example in Fig. 16 and Fig. 17, the bearing hole Hd is formed at a position at greater distances from the proximal end of the stands 632b and 632c than the bearing hole He. The bearing holes Hd and He have inner diameters greater than the diameters of the shaft portions 633d and 633e to allow the shaft portions 633d and 633e to pass and prevent the liquid from being accumulated.

The stand 632d is formed between the stands 632b and 632c so as to protrude from the body section 611.

As shown in Fig. 18, the fastener 633 is configured to be able to fasten the distal end portion 41 to prevent the distal end portion 41 placed on all or some of the stands 632a, 632b, 632c and 632d from coming off. In the fastener 633, a pressing portion 633a is formed by bending a bar-shaped member made of metal or the like at the center to press the distal end portion 41, support portions 633b and 633c parallel to each other to support the pressing portion 633a are formed by bending both end portions of the pressing portion 633a, a shaft portion 633d is formed by bending an end portion of the one support portion 633b at an obtuse angle and a shaft portion 633e is formed by bending an end portion of the other support portion 633c at an acute angle. A cylindrical slip prevention member 633f made of rubber or the like is attached to the pressing portion 633a. The shaft portion 633d is inserted into the bearing hole He. The shaft portion 633e is inserted into the bearing hole Hd.

The fastener 633 is attached to the bearing holes Hd and He while changing the positions of the shaft portions 633d and 633e by an external force. The fastener 633 is freely turnably supported by the body section 611. The amount of deformation of the fastener 633 in a laid-down state is relatively smaller than the amount of deformation in a raised state and the fastener 633 is biased to lay down by a restoring force.

As shown in Fig. 19, the first nozzle 641a and the second nozzle 641b communicate with the fluid channel R and are configured to be able to lead out the fluid introduced from the connection portion 121. The first nozzle 641a is formed in a predetermined length, to be longer than the second nozzle 641b. The first nozzle 641a and the second nozzle 641b are integrally formed so as to protrude from the body section 611 and a retainer Rt is provided between the first nozzle 641a and the second nozzle 641b.

The retainer Rt is formed into a convex shape so as to hit the forceps elevator 45 in the concave portion 42 and thereby improve holding performance of the distal end portion 41.

The fixing portion 651 is provided so as to protrude from the body section 611 in a direction opposite to the fluid leading-out direction. A mounting concave portion 652 for mounting on the holding net N is provided between the fixing portion 651 and the support piece 611b. With the holding pin P disposed in the mounting concave portion 652, the endoscope reprocessing tool 601 is mounted on the holding net N so that the holding pin P is movable within the mounting concave portion 652.

Chamfer-shaped tapered portions Tp3 are provided on both sides of the proximal end of the fixing portion 651. Two endoscope reprocessing tools 601 can be placed on the holding net N such that the tapered portions Tp3 are placed adjacent to each other.

A first opening portion 661a is provided on the stand 632a in a direction orthogonal to the fluid leading-out direction of the first fluid outlet 142a and configured so as to allow the distal end portion 41 to cross. More specifically, the first opening portion 661a is formed such that the first opening portion 661a is surrounded by the body section 611 and the stand 632a on three sides and the space surrounded by the body section 611 and the stand 632a is opened in order to allow the distal end portion 41 to be placed on the first nozzle side end portion 131a.

A second opening portion 661b is configured so as to allow the distal end portion 41 to cross. More specifically, the second opening portion 661b is formed so as to be surrounded by the stands 632b, 632c and 632d on three sides to allow the distal end portion 41 to be crossly arranged on the second nozzle side end portion 131b so that the space surrounded by the stands 632b, 632c and 632d is opened.

As shown in Fig. 20, when the insertion portion 31 is placed on the support piece 611b, the distal end portion 41 is placed on the stand 632a and the fastener 633 is laid down, the holding portion 631 holds the endoscope 1.

As shown in Fig. 21, the holding portion 631 supports both sides of the ultrasound transducer H by the tapered portions Tp2 in such a way that a vibration surface of the ultrasound transducer H does not touch the stand 632d and causes the fastener 633 to lay down to hold the distal end portion 41 with the ultrasound transducer H.

That is, the endoscope reprocessing tool 601 includes the fastener 633 to fasten the distal end portion 41 to prevent the distal end portion 41 from coming off, provides the stands 632b and 632c so as to face each other, and turnably attaches the fastener 633 to the bearing holes Hd and He provided on the stands 632b and 632c respectively. The bearing holes Hd and He are formed at positions at the same distance from the second opening portion and at positions at different distances from the respective proximal ends of the stands 632b and 632c, the fastener 633 includes a pair of the shaft portions 633d and 633e arranged at such positions facing each other, changes the positions of the shaft portions 633d and 633e by an external force so that the shaft portions 633d and 633e are inserted into the bearing holes Hd and He respectively and biased by a restoring force trying to return to the original shape in a laying-down direction.

According to the above-described third embodiment, the endoscope reprocessing tool 601 can fasten the distal end portion 41 by the fastener 633, position the concave portion 42 with respect to a plurality of the endoscopes 1, the distal end portion 41 of the insertion portion 31 of which varies in shape, lead out the fluid from the first nozzle 641a and the second nozzle 641b into the concave portion 42 and thereby perform reprocessing of the interior of the concave portion 42.

### (Modification of Third Embodiment)

In the third embodiment, the ultrasound transducer H is held by the tapered portions Tp2 so as to prevent the vibration surface of the ultrasound transducer H from touching the stand 632d, but the ultrasound transducer H may be held by a holding protrusion Pr.

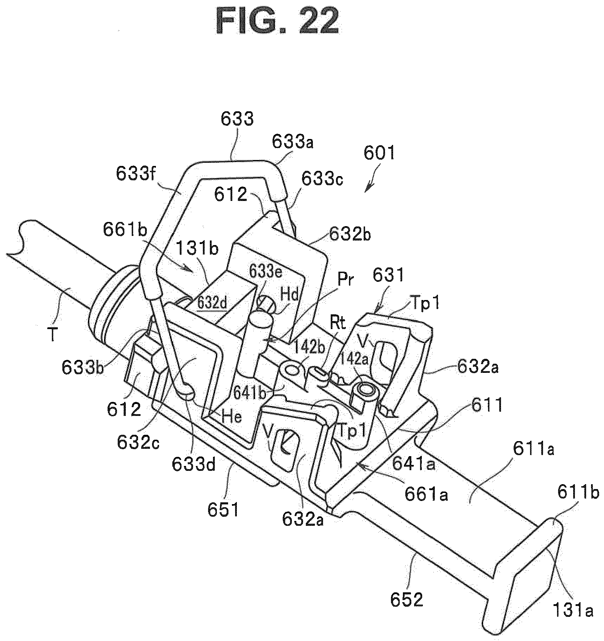

Fig. 22 is a perspective view illustrating an external appearance configuration of an endoscope reprocessing tool 601 according to a modification of the third embodiment of the present invention. In the description of the present modification, description of the same components as the components in the other embodiments and modifications will be omitted.

The endoscope reprocessing tool 601 includes the holding protrusion Pr.

The holding protrusion Pr is formed so as to protrude from the body section 611 and configured to hold the distal end portion 41. That is, the holding protrusion Pr is formed so as to protrude in the fluid leading-out direction and configured to hit a guide member and hold the distal end portion 41.

The endoscope reprocessing tool 601 can thereby hold the endoscope 1 with the ultrasound transducer H such that the vibration surface of the ultrasound transducer H does not touch the stand 632 and the connection tube T.

Note that in the embodiment, the fixing portion 151 includes the mounting hole 152, but the fixing portion 151 is not limited to the configuration including the mounting hole 152. The fixing portion 151 may be formed into, for example, a C shape or may be formed into a block shape so that the fixing portion 151 is internally fitted into the bar-shaped member of the holding net N.

Note that in the embodiment, although the fluid supply source is the endoscope reprocessor 201, the fluid supply source may be an external pump which is not shown.

Note that in the embodiment, two stands 132 are provided, but the number of stands 132 may be one or three or more.

Note that in the embodiment, although two stopper portions 133 are provided, the number of stopper portions 133 may be one or three or more.

The present invention is not limited to the aforementioned embodiments, but various changes or alterations may be made within the scope not changing the gist of the present invention.

According to the present invention, it is possible to provide an endoscope reprocessing tool capable of positioning a concave portion with respect to a plurality of endoscopes, a distal end portion of an insertion portion of which varies in shape, leading out a fluid from a nozzle into the concave portion and performing reprocessing of the interior of the concave portion.

The present application claims priority based on Japanese Patent Application No. 2016-087183, filed on April 25, 2016, the disclosure of which is incorporated in the specification, claims and drawings of the present application by reference.

## Claims

1. An endoscope reprocessing tool comprising:
a connection portion connected to a fluid supply source;
a first fluid outlet configured to communicate with the connection portion and lead out a fluid;
one or a plurality of stands arranged parallel to a fluid leading-out direction of the first fluid outlet, provided with a guide member to guide a treatment instrument to a concave portion and configured to place a distal end portion of an endoscope;
a first opening portion provided at one end of the stand and configured to allow the distal end portion to cross;
a second opening portion provided at another end of the stand and configured to allow the distal end portion to cross; and
a positioning portion configured to hit a proximal end side wall of the concave portion and position the distal end portion when the distal end portion is placed on the stand such that a side of the first opening portion is on a proximal end and a side of the second opening portion is on a distal end.

2. The endoscope reprocessing tool according to claim 1, further comprising a second fluid outlet disposed closer to the second opening portion side than the first fluid outlet and configured to lead out the fluid.

3. The endoscope reprocessing tool according to claim 1, wherein the positioning portion is a first nozzle, at a distal end of which the first fluid outlet is formed.

4. The endoscope reprocessing tool according to claim 1, further comprising a fixing portion configured to fix the endoscope reprocessing tool to a treatment tank of an endoscope reprocessor or to a holding net disposed in the treatment tank.

5. The endoscope reprocessing tool according to claim 1, further comprising one or a plurality of stopper portions configured to prevent the distal end portion from coming off, wherein
the stopper portion is provided on the stand so as to cross the fluid leading-out direction of the first fluid outlet.

6. The endoscope reprocessing tool according to claim 5, wherein the first opening portion and the second opening portion are formed so that a space surrounded by the stopper portion, the stand and the body section on three sides is opened.

7. The endoscope reprocessing tool according to claim 1, further comprising one or a plurality of stopper portions configured to prevent the distal end portion from coming off, wherein
the stopper portion is freely openable/closable with respect to the stand.

8. The endoscope reprocessing tool according to claim 7, wherein the stopper portion is freely turnable around a shaft part provided in a direction crossing a direction along the fluid leading-out direction.

9. The endoscope reprocessing tool according to claim 1, further comprising a fastener configured to fasten the distal end portion so as not to come off, wherein
the stands are provided so as to face each other, and
the fastener is freely turnably attached to respective bearing holes provided on the stands.

10. The endoscope reprocessing tool according to claim 9, wherein
the bearing holes are formed at positions at a same distance from the second opening portion and at positions at different distances from the respective proximal ends of the stands, and
the fastener comprises a pair of shaft portions disposed at positions facing each other and the positions of the shaft portions are changed by an external force so that the shaft portions are inserted into the bearing holes and biased toward a laying-down direction by a restoring force trying to return to an original shape.

11. The endoscope reprocessing tool according to claim 1, further comprising a holding protrusion formed so as to protrude in the fluid leading-out direction and configured to hit the guide member and hold the distal end portion.
